# EUROPEAN PATENT APPLICATION

(11) **EP 2 606 832 A2**
(43) Date of publication of application: **26.06.2013**
(21) Application number: 12198013.0
(22) Date of filing: 19.12.2012
(51) Int. Cl.: A61B 17/04

(54) **Continuous suture passing instrument**

(30) Priority: 22.12.2011 US 201161579218 P
(71) Applicant: Arthrex, Inc., Naples, FL 34108-1945 (US)
(72) Inventor: Dreyfuss, Peter J., Naples, Florida 34112 (US); Chudik, Steven C., Westmont, Illinois 60559 (US)
(74) Representative: Leihkauf, Steffen Falk

(57) **Abstract**

A suture passing device that may be used in both open and endoscopic surgical procedures and that are designed to continuously deploy suture. The surgical device is provided with a handle assembly including an actuator in communication with a pair of advancement wheels and a built-in spool of suture (with a long length of material that can be fed through tissue and cut at desired locations). By moving the actuator linearly, the advancement wheels rotate, causing the spool to rotate and advance the suture through a shaft of the instrument to the tissue to be sutured, in a continuous manner.

## Description

### FIELD OF THE INVENTION

The present invention relates to surgical devices and, more particularly, to a surgical instrument for suturing of tissue during surgery.

### BACKGROUND OF THE INVENTION

Surgical procedures often require sutures to ligate, join or otherwise treat tissue. Generally, suture needles with attached suture strands are grasped either manually or by forceps and passed through the desired work site so a knot can be tied. While the procedures are fairly uncomplicated in open surgery where most suture sites are readily accessible, in endoscopic procedures, where access to the work site is not readily available, the surgeon must use auxiliary devices to grasp the suture strands and pass them through desired tissue.

Various instruments and techniques have been developed and are known for surgical repairs requiring the passing of sutures to distant locations. There is a need, however, for an improved device and technique that enables the surgeon to pass sutures arthroscopically to an internal body part where the work site is only accessible through a small portal or cannula and it is difficult to pass sutures within the body.

Further, depending on the location of the damage and the extent of the damage, a considerable length of suture may need to be employed to complete the repair. For example, when repairing the glenohumeral joint during endoscopic surgery as much as 9 inches of suture or wire will need to be advanced. In addition, reloading of suture passing instruments such as Lasso-type instruments is difficult as the user (the surgeon or the support staff) needs to constantly pull the instrument out of the surgical site and reload it with the necessary amount of suture.

What is needed is a device with a deployment mechanism that will reduce the time and effort needed to advance a lengthy suture. A suture passing instrument that is always loaded with suture so that the user does not need to reload the instrument is also needed. A device with a long length of material that can be fed through tissue and cut at desired locations is also needed.

### SUMMARY OF THE INVENTION

The present invention provides a suture passing device that may be used in both open and endoscopic surgical procedures and that is designed with a deployment mechanism that deploys a suture or flexible material in a continuous manner so that the device is ready to use again immediately. The instrument is always loaded with flexible material (suture) so that the user (surgeon) does not have to reload the instrument. The suture (flexible material) is passed without the need to pull the instrument out of the surgical site to reload the suture.

The suture passing device is provided with a handle having a coil of flexible material (suture) located at its most proximal end and a pair of advancement wheels for advancing the flexible material. By rotating the advancement wheels (with a user's thumb, for example), the flexible material is drawn from the coil and advanced through a shaft of the instrument to the tissue to be sutured. The coil of flexible material is provided integral with the handle and within the handle, the coil being wrapped around a supporting structure of the handle before assembling the suture passing instrument. The supporting structure may include a rotatable spool built in within the handle, or post(s) in the handle, all structures being integral with the handle. The device may be optionally provided with a cutting mechanism (for example, a cutting blade).

These and other features and advantages will be more apparent from the following detailed description that is provided in connection with the accompanying drawing and illustrated exemplary embodiments.

### BRIEF DESCRIPTION OF THE DRAWINGS

FIG. 1 illustrates a partial, internal side view of a suture passing device of the present invention (with a rotatable spool built into the handle).

FIG. 2 illustrates another partial, internal side view of the suture passing device of the present invention.

FIG. 3 illustrates another partial, internal side view of the suture passing device of the present invention.

FIG. 4 illustrates a schematic side view of another suture passing device of the present invention (showing the suture and a cutting mechanism integral with the instrument).

FIG. 5 illustrates a partial, internal side view of yet another suture passing device of the present invention (with a post built into the handle).

### DETAILED DESCRIPTION OF THE PREFERRED EMBODIMENTS

The present invention provides a suture passing instrument for use in both open and endoscopic surgical procedures that is designed to incorporate a supporting structure (a spool, cartridge and/or posts) for deploying sutures or flexible materials in a continuous manner.

In one exemplary embodiment, the surgical instrument is provided with a handle assembly located at a distal end and includes a rotatable spool (or cartridge) of flexible material (suture) located within the handle assembly. The spool is preferably built in (within) the handle and is integral with the handle. The free end of the flexible material (suture) is fed through a pair of advancement wheels at the proximal end of the handle. By rotating the advancement wheels, with the user's thumb, for example, the suture is advanced continuously from the spool, through a cannulated shaft of the instrument and to the tissue to be sutured. By providing a spool of suture within the handle, the instrument is always loaded with suture so the user (surgeon) does not need to reload the instrument.

In an additional embodiment, instead of storing the suture (flexible material) on a rotatable spool, the suture (flexible material) can simply be wrapped around a structure integral with the handle (built into the handle), such as post(s) built into the handle, for example, before assembling the instrument.

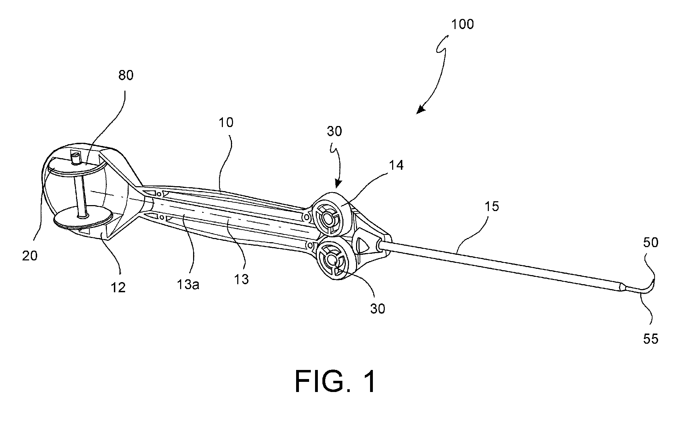

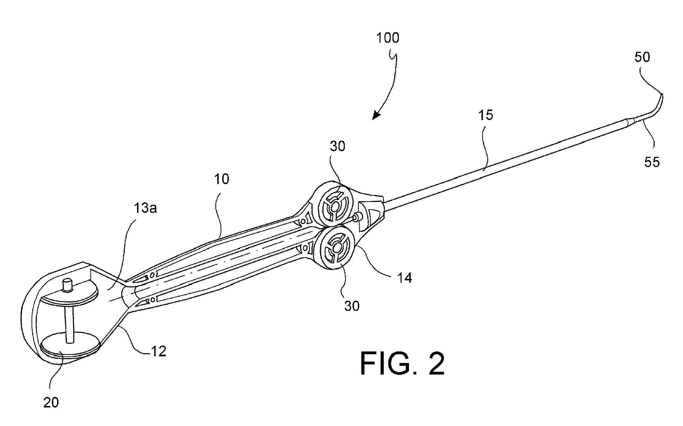

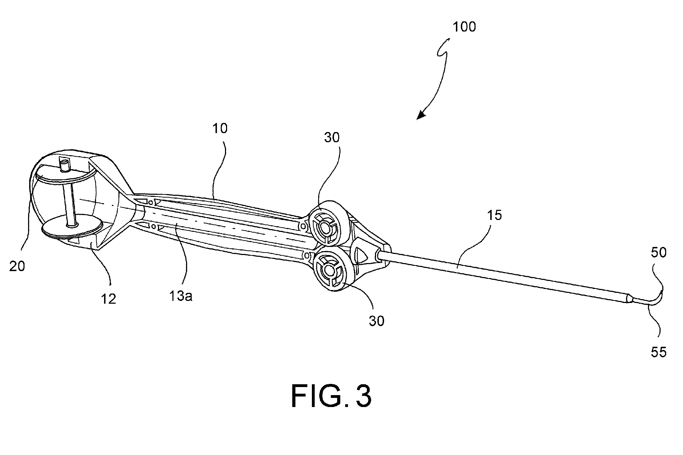

Referring now to the drawings, where like elements are designated by like reference numerals, FIGS. 1-5 illustrate suture passing device 100, 200, 300. Device 100, 200, 300 may be a Lasso-type instrument (such as Arthrex SutureLasso™, for example) and includes a handle assembly 10 with a built-in supporting structure 80 that supports a coil 66 of flexible material 60 (suture 60) that is deployed (advanced) continuously from the coil and supporting structure, through a cannulated shaft of the instrument, and to the tissue to be sutured. By providing a coil of suture within the handle, the instrument is always loaded with suture so the user (surgeon) does not need to reload the instrument.

The handle assembly 10 has a proximal end 12 and a distal end 14, a cannulation 13 extending from the proximal end to the distal end, a supporting structure 80 located at the proximal end 12, and an actuator 30 located at the distal end 14. Device 100 further includes cannulated shaft 15 that extends away from proximal end 12 and terminates in a substantially curved distal end 55 with sharp tip 50.

As detailed below, supporting structure 80 is preferably in the form of a built-in spool or cartridge or other similar structures such as posts embedded into the handle, and that allows a coil of material strand to be wrapped around it and be deployed for suturing. Supporting structure 80 is provided at the proximal end 12 of the handle 10 and extends about perpendicular to longitudinal axis 13a of handle 10.

As also detailed below, actuator 30 is configured to advance (move) a flexible strand (suture) 60 of coil 66 through cannulation 13 of the handle and through the cannulated shaft 15 and out of the substantially curved distal end 55 and sharp tip 50.

In an exemplary embodiment and as shown in FIGS. 1-5, device 100, 200, 300 is a modified SutureLasso™ instrument such as the one described and detailed in U.S. Publ. No. 2005/0033365, entitled "Cannulated Instrument with Curved Shaft for Passing Suture through Tissue." As detailed in U.S. Publ. No. 2005/0033365, the SutureLasso is a single use instrument packaged sterile having a curved tip configuration for arthroscopic Bankart, SLAP and rotator cuff repairs, among others. The tip may have a diameter between 1.8 mm to about 2.3 mm outer diameter tip. The sharp tip and small diameter shaft will easily penetrate soft tissue while the reinforced shaft resists bending.

In an exemplary embodiment, instrument (device) 100 is a modified SutureLasso™ -type instrument provided with a supporting structure 80 in the form of a built-in spool 20 which comes preloaded with an amount of flexible strand or flexible material 60 (suture such as monofilament or braided suture) forming coil 66. Preferably, coil 66 is provided onto the spool (cartridge) 20 before the final assembly of the instrument.

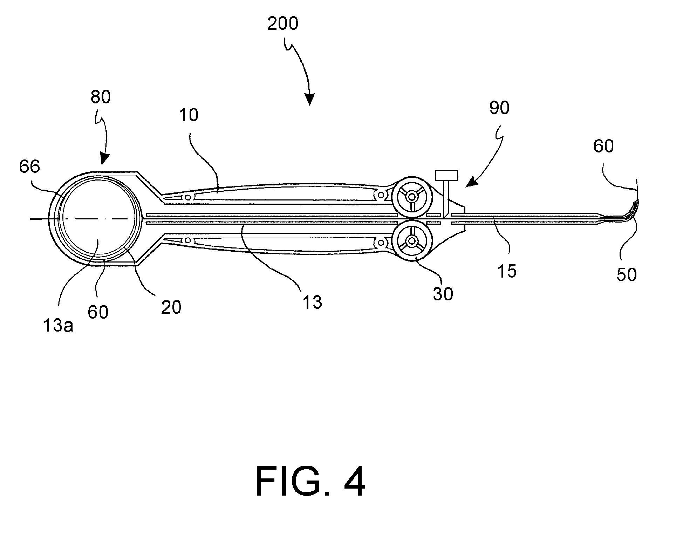

Device 100 operates to advance flexible material 60 (FIG. 4) from coil 66 through cannulated shaft 15 for engagement with desired tissue. Actuating the actuator 30 (pair of advancement wheels 30) allows the flexible material (suture) 60 to pass through the cannulated handle 10 and through the shaft 15 and out of the sharp tip 50.

Flexible material 60 may be suture (for example, a monofilament or braided suture) or similar materials. Flexible material 60 is advanced when an operator rotates advancement wheels 30, located near the distal end of handle assembly 10, using a thumb, for example. By storing the flexible material 60 on a spool 20 (cartridge 20) in the handle, the length of flexible material 60 is advanced in a continuous manner through cannulated tube 15 toward the tissue to be sutured without the need of the user to pull the instrument and reload the flexible material (suture).

The suture passing instrument may be optionally provided with a cutting mechanism 90 which may be, for example, a cutting blade such as a built-in blade, among others. However, the suture passing instrument need not be provided with a cutting mechanism, in which case the flexible material (suture) may be cut off manually at the tip of the instrument by the user, subsequent to the user withdrawing the suture passer (the modified SutureLasso™) after feeding through the desired amount of suture, and without the need to feed up another "x" amount of suture before the next throw.

FIG. 4 illustrates an exemplary suture passing device 200 which is similar to device 100 of FIGS. 1-3 but differs in that device 200 is provided with a cutting mechanism 90 which may be, for example, a cutting blade such as a built-in blade. The cutting mechanism is provided on the handle and located between the actuator 30 (advancement wheels 30) and proximal end of cannulated shaft 15.

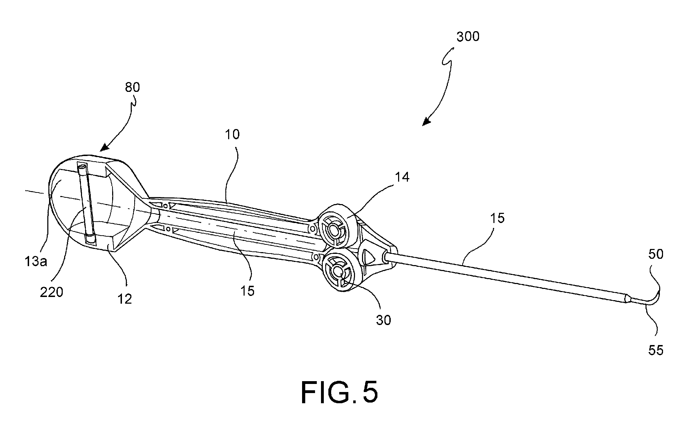

In yet another embodiment and as shown in FIG. 5, instrument (suture passing device) 300 is a modified SutureLasso™ -type instrument provided without an actual spool of suture, i.e., without built-in spool 20. According to this embodiment, the instrument 300 has coil 66 with an amount of flexible strand 60 (suture 60) wrapped around supporting structure 80 which is in the form of at least one post 220. The post 220 is provided in the handle (integral with the handle) and the coil 66 is provided around the post before assembling the instrument. The post 220 is preferably manufactured with the instrument by known methods in the art such as insert molding, for example, and the coil 66 is provided onto the post before the final assembly of the instrument 300.

In all the above described embodiments, suture 60 for use in device 100, 200, 300 may be of any configuration and may comprise any type of material, including suture loops, FiberTape®, FiberWire®, shuttle sutures, suturing sutures, among many others. Suture 60 may be a monofilament or a braided suture, for example, a stiff braided suture such as #2 FiberStick™.

The suture passing instrument 100, 200, 300 is always loaded with flexible material 60 (suture 60) so that the user (surgeon) does not have to reload the instrument. The suture 60 (flexible material 60) is passed without the need to pull the instrument 100, 200 out of the surgical site to reload the suture.

While the present embodiments are described herein with reference to illustrative figures for particular applications, it should be understood that the embodiments are not limited thereto. Those having ordinary skill in the art and access to the teachings provided herein will recognize additional modifications, applications, embodiments and substitution of equivalents all fall within the scope of the presented embodiments. Accordingly, the embodiments are not to be considered as limited by the foregoing description.

loop of suture used to shuttle suture through soft tissue. The Small Diameter Lasso has a 1.8 mm tip diameter and comes preloaded with a Nitinol Wire Loop used to shuttle suture through soft tissue. The QuickPass™ Lasso has the same dimensions as the Small Diameter Lasso and features thumb wheels to advance the Nitinol Wire Loop. Both of these instruments can also be loaded with a monofilament suture for shuttling or with #2 FiberStick™ to pass a stitch when using the PushLock® or SwiveLock® anchors eliminating the shuttle step.

After the tip is passed through soft tissue, the loop is deployed and retrieved through a cannula with a Crochet Hook or Suture Retriever. A suture strand or suture tail from a previously placed suture anchor is placed in the loop, and the opposite end of the loop is pulled, delivering the suture through tissue and out the cannula.

## Claims

1. A suture passer instrument, comprising:
a cannulated shaft having a proximal end and a substantially curved distal end;
a cannulated handle coupled to the proximal end of the cannulated shaft;
a supporting structure integral with the handle and located at a proximal end of the handle;
a flexible strand wrapped around the supporting structure and extending through the cannulated handle and through the shaft; and
an actuator provided within the handle, the actuator being configured to advance the flexible strand from the supporting structure, through the handle and through the cannulated shaft, and out of the substantially curved distal end.

2. The suture passer instrument of claim 1, wherein the supporting structure is a spool built into the handle or a post built into the handle.

3. The suture passer instrument of claim 1, wherein the supporting structure is insert molded into the handle and the flexible strand is wrapped around the supporting structure before assembling of the instrument.

4. The suture passer instrument of claim 1, wherein the cannulated shaft has a substantially curved region, the substantially curved region having a configuration designed for insertion percutaneously through the rotator cuff, medially to laterally, through the Modified Neviaser Portal.

5. The suture passer instrument of claim 1, wherein the substantially curved distal end terminates in a sharp tip.

6. The suture passer instrument of claim 1, wherein the actuator includes a pair of advancement wheels that are actuated by a user's thumb.

7. The suture passer instrument of claim 1, wherein the substantially curved distal end has a corkscrew configuration.

8. The suture passer instrument of claim 1, wherein the flexible strand is a monofilament or a braided suture.

9. The suture passer instrument of claim 1, further comprising a cutting mechanism integral with the instrument for cutting the flexible strand.
